# FASCICULE DE BREVET EUROPEEN

(11) **EP 0 852 489 B1**
(45) Date de publication et mention de la délivrance du brevet: **08.05.2002**
(21) Numéro de dépôt: 96931858.3
(22) Date de dépôt: 16.09.1996
(51) Int. Cl.: A61K 7/48, A61K 7/06

(54) **COMPOSITION CAPILLAIRE COMPRENANT AU MOINS UN POLYMERE SILICONE GREFFE PAR DES MONOMERES ANIONIQUES, AMPHOTERES OU NON-IONIQUES ET AU MOINS UN POLYMERE AMPHOTERE**
ZUSAMMENSETZUNG FÜR DAS HAAR, DIE MINDESTENS EIN MIT ANIONISCHEN, AMPHOTEREN ODER NICHTIONISCHEN MONOMEREN GEPFROPFTES SILICONPOLYMER UND MINDESTENS EIN AMPHOTERES POLYMER ENTHÄLT
HAIR CARE COMPOSITION INCLUDING AT LEAST ONE SILICONE POLYMER GRAFTED BY ANIONIC, AMPHOTERIC OR NON-IONIC MONOMERS, AND AT LEAST ONE AMPHOTERIC POLYMER

(30) Priorité: 29.09.1995 FR 9511488
(43) Date de publication de la demande: 15.07.1998
(73) Titulaire: L'OREAL, 75008 Paris (FR)
(72) Inventeur: DUBIEF, Claude, F-78150 Le Chesnay (FR); DUPUIS, Christine, F-75018 Paris (FR); CAUWET-MARTIN, Danièle, F-75011 Paris (FR)
(74) Mandataire: Le Blainvaux Bellegarde, Françoise
(86) Numéro de dépôt international: FR9601440
(87) Numéro de publication internationale: WO9712596

(56) Documents cités:
- EP-A- 0 582 152
- WO-A-91/15186
- WO-A-95/00108
- FR-A- 2 709 955

## Description

La présente invention a trait à une composition cosmétique ou dermatologique pour le traitement des matières kératiniques, en particulier des cheveux humains comprenant au moins un polymère siliconé greffé, à squelette polysiloxanique greffé par des monomères organiques non-siliconés, anionique, amphotère ou non-ionique et au moins un polymère amphotère.

On connaît dans l'état de la technique des polymères siliconés greffés tels que ceux décrits dans les demandes de brevet EP-A-0582152 et WO 93/23009. Ces polymères sont proposés dans les compositions capillaires pour leurs propriétés coiffantes. Cependant, lorsque l'on utilise ces polymères, la tenue de la coiffure et le toucher des cheveux ne sont pas satisfaisants.

La demanderesse a découvert de façon surprenante qu'en associant au moins un polymère siliconé greffé du type anionique, amphotère ou non-ionique avec au moins un polymère amphotère dans un rapport polymère amphotère/polymère siliconé greffé compris entre 0,25 et 15, on obtenait des propriétés de toucher et de douceur des cheveux sensiblement supérieures à celles obtenues avec chaque polymère utilisé seul.

La composition selon l'invention est donc essentiellement caractérisée par le fait qu'elle comprend dans un milieu cosmétiquement ou dermatologiquement acceptable au moins un polymère siliconé greffé, à squelette polysiloxanique greffé par des monomères organiques non-siliconés, du type anionique, amphotère ou non-ionique et au moins un polymère amphotère dans un rapport polymère amphotère/polymère siliconé greffé compris entre 0,25 et 15.

Dans ce qui suit, on entend désigner par polymère siliconé, en conformité avec l'acceptation générale, tout polymère ou oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et én particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements amides, les radicaux acyloxy ou acyloxyalkyls, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon la présente invention, le ou les polymères siliconés qui doivent être utilisés sont ceux qui comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Ces polymères siliconés peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques CH₂=CH-, ou encore la réaction entre des groupements thio-fonctionnels -SH avec ces mêmes groupements vinyliques.

Des exemples de polymères siliconés convenant à la mise en oeuvre de la présente invention, ainsi que leur mode particulier de préparation, sont notamment décrits dans les demandes de brevets EP-A-0582152, WO 93/23009 et WO 95/03776.

Selon un mode particulièrement préféré de réalisation de la présente invention, le polymère siliconé mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélanges, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrylate d'isooctyle, le (méth)acrylate d'isonyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.

Une famille de polymères siliconés greffés, particulièrement bien à la mise en oeuvre de la présente invention est constituée par les polymères siliconés comportant dans leur structure le motif de formule (I) suivant : 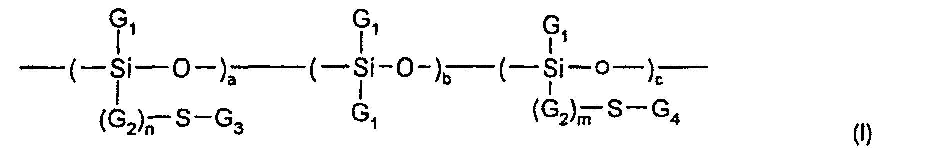 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀; G₃ représente un reste polymérique résultant de l'(homo)polymérisation d'au moins un monomère anionique à insaturation éthylénique ; G₄ représente un reste polymérique résultant de l'(homo)-polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de O et 50 ; sous réserve que l'un des paramètres a et c soit différent de 0.

De préférence, le motif de formule (I) ci-dessus présente au moins l'une, et encore plus préférentiellement l'ensemble, des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle(C₁-C₁₀), de préférence du type (méth)acrylate d'isobutyle ou de méthyle.

Des exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

D'autres exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés de l'invention varie de 10 000 à 1 000 000 environ, et encore plus préférentiellement de 10 000 à 100 000 environ.

Le polymère siliconé greffé est utilisé de préférence en une quantité allant de 0,01 à 20% en poids du poids total de la composition. Encore plus préférentiellement, cette quantité varie de 0,1 à 15% en poids et encore plus particulièrement de 0,5 à 10% en poids.

Selon l'invention, on peut utiliser tout polymère amphotère connu en soi. Ces polymères sont de préférence des polymères fixants, c'est à dire ayant pour fonction de fixer temporairement la forme de la coiffure.

Bien entendu, on peut utiliser un ou plusieurs polymères amphotères.

Les polymères amphotères utilisables conformément à l'invention peuvent être choisis parmi les polymères comportant des motifs A et B répartis statistiquement dans la chaîne polymère où A désigne un motif dérivant d'un monomère comportant au moins un atome d'azote basique et B désigne un motif dérivant d'un monomère acide comportant un ou plusieurs groupements carboxyliques ou sulfoniques ou bien A et B peuvent désigner des groupements dérivant de monomères zwittérioniques de carboxybétaïnes ou de sulfobétaïnes ;
A et B peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien A et B font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères dérivés de diallyldialkylammonium et d'au moins un monomère anionique tels que les polymères comportant environ 60 à environ 99% en poids d'unités dérivés d'un monomère de diallyldialkylammonium quatemaire dans lequel les groupements alkyle sont choisis indépendamment parmi les groupements alkyle ayant 1 à 18 atomes de carbone et dans lequel l'anion est dérivé d'un acide ayant une constante d'ionisation supérieure à 10⁻¹³ et 1 à 40% en poids de ce polymère, d'un monomère anionique choisi parmi les acides acrylique ou méthacrylique, le poids moléculaire de ce polymère étant compris entre environ 50.000 et 10.000.000 déterminé par chromatographie par perméation de gel. De tels polymères sont décrits dans la demande EP-A-269243.
   Les polymères préférés sont entre autres les polymères comportant des groupements alkyle choisis parmi les groupements ayant 1 à 4 atomes de carbone et plus particulièrement des groupements méthyle, éthyle.
   Parmi ces polymères, les copolymères de chlorure de diméthyldiallylammonium ou de diéthyldiallylammonium et d'acide acrylique sont particulièrement préférés. Ces polymères sont par exemple vendus sous les dénominations "MERQUAT 280" et "MERQUAT 295" par la société MERCK.
   On peut également utiliser les terpolymères de chlorure de diméthyldiallylammonium/acide acrylique/acrylamide vendu sous la dénomination "MERQUAT PLUS 3330" par la société MERCK.
(3) les polymères comportant des motifs dérivant
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) au moins un comonomère basique tel que des esters à substituants amine primaire, secondaire, tertiaire et quaternaire des acides acrylique et méthacrylique et le produit de quatemisation du méthacrylate de diméthylaminoéthyle avec le sulfate de diméthyle ou diéthyle.

   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atome de carbone des acides ou des anhydrides maléïque ou fumarique.
   Les comonomères basiques préférés sont des méthacrylates d'aminoéthyle, de butyl aminoéthyle, de N,N'-diméthylaminoéthyle, de N-terrio-butylaminoéthyle.
   On utilise particulièrement les copolymères dont la dénomination CTFA (4ème Ed., 1991) est Octylacrylamidelacrylates/butylaminoethylmethacrylate copolymer tels que les produits vendus sous la dénomination AMPHOMER ou LOVOCRYL 47 par la société NATIONAL STARCH.
(4) les polyamino amides réticulés et alcoylés partiellement ou totalement dérivant de poyaminoamides de formule générale : 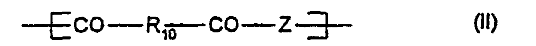 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bisprimaire, mono ou bis-secondaire et de préférence représente :
   a) dans les proportions de 60 à 100 moles %, le radical 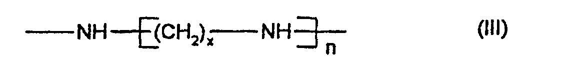 où x=2 et n=2 ou 3 ou bien x=3 et n=2 ce radical dérivant de la diéthylène triamine, de la triéthylène tétraamine ou de la dipropylène triamine ;
   b) dans les proportions de 0 à 40 moles % le radical (III) ci-dessus, dans lequel x=2 et n=1 et qui dérive de l'éthylènediamine, ou le radical dérivant de la pipérazine : 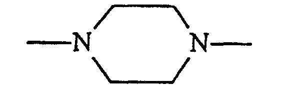
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

   Les acides carboxyliques saturés sont choisis de préférence parmi les acides ayant 6 à 10 atomes de carbone tels que l'acide adipique, triméthyl-2,2,4- et -2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(5) les polymères comportant des motifs zwittérioniques de formule : 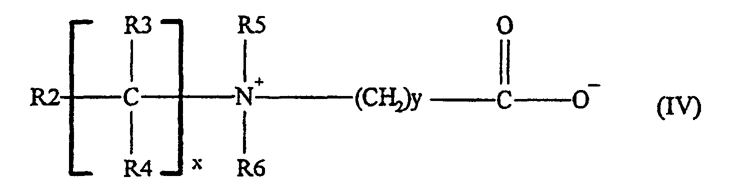 dans laquelle R₂ désigne un groupement insaturé polymérisable tel qu'un groupement acrylate, méthacrylate, acrylamide ou méthacrylamide, x et y représente un nombre entier de 1 à 3, R₃ et R₄ représentent hydrogène, méthyle, éthyle ou propyle, R₅ et R₆ représentent un atome d'hydrogène ou un radical alkyle de telle façon que la somme des atomes de carbone dans R₅ et R₆ ne dépasse pas 10.
   Les polymères comprenant de telles unités peuvent également comporter des motifs dérivés de monomères non zwittérioniques tels que la vinylpyrrolidone, l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de méthyle / diméthyl carboxyméthylammonio éthylméthacrylate de méthyle.
(6) les polymères dérivés du chitosane comportant des motifs monomères répondant aux formules suivantes : 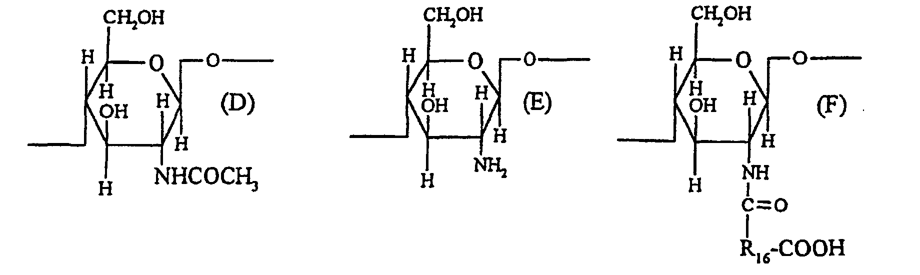 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 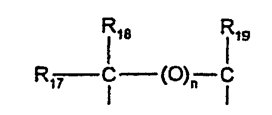 dans laquelle si n=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialkylamine éventuellement interrompus par un ou plusieurs atomes d'azote et/ou éventuellement substitués par un ou plusieurs groupes amine, hydroxyle, carboxyle, akylthio, sulfonique, un reste alcoylthio dont le groupe alcoyle porte un reste amino, l'un au moins des radicaux R₁₇, R₁₈ et R₁₉ étant dans ce cas un atome d'hydrogène ;
   ou si n=1, R₁₇, R₁₈ et R₁₉ représentent chacun un atome d'hydrogène, ainsi que les sels formés par ces composés avec des bases ou des acides.
(7) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(8) Les polymères répondant à la formule générale (V) sont décrits dans le brevet français 1 400 366 : 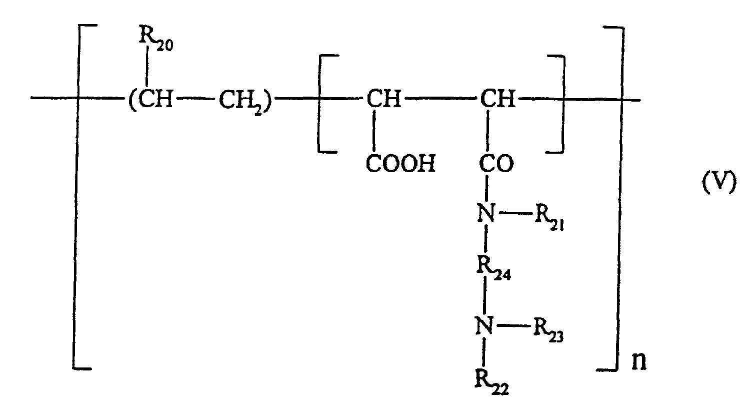 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂- , -CH₂-CH₂-CH₂- , -CH₂-CH(CH₃)-, R₂₂ ayant les significations mentionnées ci-dessus,
   ainsi que les homologues supérieurs de ces radicaux et contenant jusqu'à 6 atomes de carbone.
(9) les polymères amphotères du type -A-Z-A-Z choisis parmi:
   a) les polymères obtenus par action de l'acide chloracétique ou le chloracétate de sodium sur les composés comportant au moins un motif de formule :

      -A-Z-A-Z-A- (VI)

      où A désigne un radical 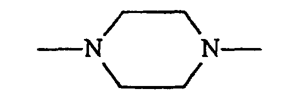 et Z désigne le symbole B ou B', B ou B' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quatemaire, amide, imide, alcool, ester et/ou uréthanne.
   b) Les polymères de formule :

      -A-Z-A-Z- (VI')
   où A désigne un radical 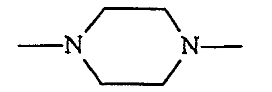 et Z désigne le symbole B ou B' et au moins une fois B'; B ayant la signification indiquée ci-dessus et B' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonction carboxyle ou une ou plusieurs fonctions hydroxyls et bétaïnisées par réaction avec l'acide chloracétique ou du chloracétate de soude.
(10) les copolymères alkyl (C₁-C₅) vinyléther/anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (3) tels que les copolymères dont la dénomination CTFA est octylacrylamide/- acrylates/butylaminoethylmethacrylate copolymer tels que les produits vendus sous la dénomination AMPHOMER ou LOVOCRYL 47 par la société NATIONAL STARCH et ceux de la famille (5) tels que le copolymère méthacryloxyéthyl N,N-diméthyl carboxyméthylbétaïne/méthacrylate de butyle vendu sous la dénomination YUKAFORMER AM 75 par la société MITSUBISHI.

Selon l'invention, on peut également utiliser les polymères amphotères sous forme de latex ou de pseudolatex, c'est-à-dire sous forme d'une dispersion aqueuse de particules de polymères insolubles.

Le rapport (polymère amphotère)/(polymère siliconé greffé) est de préférence compris entre 0,3 et 8.

Selon l'invention, le ou les polymères amphotères peuvent représenter de 0,1 % à 20 % en poids, de préférence de 0,2 % à 15 % en poids, et encore plus préférentiellement de 0,5 % à 10 % en poids, du poids total de la composition finale.

Le milieu cosmétiquement ou dermatologiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Les polymères siliconés greffés selon l'invention peuvent être dissous dans ledit milieu cosmétiquement acceptable ou utilisés sous forme de dispersion aqueuse de particules.

La composition de l'invention peut également contenir au moins un additif choisi parmi les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion plus ou moins épaissie ou de mousse.

Les compositions selon l'invention sont utilisées comme produits rincés ou comme produits non-rincés notamment pour le lavage, le soin, le conditionnement des matières kératiniques telles que les cheveux humains, le maintien de la coiffure ou la mise en forme de la coiffure.

Elles sont plus particulièrement des lotions de mise en plis, des lotions pour le brushing, des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également des shampooings, des compositions à rincer ou non, à appliquer avant ou après un shampooing, une coloration, une décoloration, une permanente ou un défrisage.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

L'invention a encore pour objet un procédé de traitement des matières kératiniques telles que les cheveux humains consistant à appliquer sur celles-ci une composition telle que définie précédemment puis à effectuer éventuellement un rinçage à l'eau.

L'invention va être maintenant plus complètement illustrée à l'aide des exemples suivants qui ne sauraient être considérés comme la limitant aux modes de réalisation décrits.

### EXEMPLES

### EXEMPLE 1 Spray de coiffage en flacon pompe

- Polymère siliconé greffé de formule (I) de structure polyméthyl/méthylsiloxane à groupements propyl thio-3 polyméthacrylate d'isobutyle en solution dans une silicone volatile cyclique 3 g MA
- Copolymère acide acrylique / méthacrylate de méthyle / N-tertiobutyl aminoéthyl méthacrylate / N-octylacrylamide /méthacrylate d'hydroxypropyle (AMPHOMER LV 71 de NATIONAL STARCH) 4 g MA
- Aminométhylpropanol neutralisation à 100% du polymère siliconé qsp
- Ethanol qsp 100 g

### EXEMPLE 2 Shampooing

- Polymère siliconé greffé de formule (I) de structure polyméthyl/méthylsiloxane à groupement propyl thio-3 poly(acide méthacrylique) et à groupements propyl thio-3 polyméthacrylate de méthyle 1 g MA
- Copolymère méthacryloxyéthyl-N,Ndiméthyl carboxy méthylbétaïne/méthacrylate de butyle vendu sous la dénomination YUKAFORMER AM 75 par la société MITSUBISHI 1,5g MA
- Monosulfosuccinate d'alcool laurique (C₁₂/C₁₄ ; 70/30) oxyéthyléné à 3 moles d'oxyde d'éthylène sous forme de sel disodique en solution aqueuse à 40% vendu sous le nom SETACIN 103 SPECIAL parla société ZSCHIMMEN SCHWARZ 12 g MA
- Mélange cocoyl amidopropyl bétaïne/monolaurate de glycérol (25/5) en solution aqueuse à 30% 8 g MA
- Oléfine sulfonate de sodium vendu sous le nom HOSTAPUR OS par la société HOECHST 8 g MA
- Séquestrant, parfum, conservateur qs
- NaOH qs pH 7,7
- Eau qsp 100 g

## Revendications

1. Composition cosmétique ou dermatologique destinée au traitement des matières kératiniques, **caractérisée par le fait qu'**elle comprend dans un milieu cosmétiquement ou dermatologiquement acceptable au moins un polymère siliconé greffé, à squelette polysiloxanique greffé par des monomères organiques non-siliconés, du type anionique, amphotère ou non-ionique et au moins un polymère amphotère dans un rapport (polymère amphotère)/(polymère siliconé greffé) compris entre 0,25 et 15.

2. Composition selon la revendication 1, **caractérisée par le fait que** le polymère siliconé greffé comprend une chaîne principale de polysiloxane sur laquelle se trouve greffé, à l'intérieur de ladite chaîne ainsi qu'éventuellement à l'une au moins de ses extrémités, au moins un groupement organique ne comportant pas de silicone.

3. Composition selon la revendication 1 ou 2, **caractérisée par le fait que** le polymère siliconé greffé est susceptible d'être obtenu par copolymérisation radicalaire entre d'une part au moins un monomère organique anionique non-siliconé présentant une insaturation éthylénique et/ou un monomère organique hydrophobe non-siliconé présentant une insaturation éthylénique et d'autre part un polysiloxane présentant dans sa chaîne au moins un groupement fonctionnel capable de venir réagir sur lesdites insaturations éthyléniques desdits monomères non-siliconés.

4. Composition selon la revendication 3, **caractérisée par le fait que** le monomère organique anionique à insaturation éthylénique est choisi, seul ou sous forme de mélange de monomères, parmi les acides carboxyliques insaturés, linéaires ou ramifiés.

5. Composition selon la revendication 4, **caractérisée par le fait que** le monomère organique anionique à insaturation éthylénique est choisi, seul ou sous forme de mélange de monomères, parmi l'acide acrylique, l'acide méthacrylique, l'acide maléique, l'anhydride maléique, l'acide itaconique, l'acide fumarique et l'acide crotonique ou leurs sels d'alcalins, d'alcalino-terreux ou d'ammonium, ou leurs mélanges.

6. Composition selon la revendication 3, **caractérisée par le fait que** le monomère organique hydrophobe à insaturation éthylénique est choisi, seul ou en mélange de monomères, parmi les esters d'acide acrylique d'alcanol et/ou les esters d'acide méthacrylique d'alcanol, de préférence l'alcanol étant en C₁-C₁₈.

7. Composition selon la revendication 6, **caractérisée par le fait que** le monomère organique hydrophobe à insaturation éthylénique est choisi, seul ou en mélange de monomères dans le groupe constitué par le (méth)acrylate d'isooctyle, le (méth) acrylate d'isonyle, le 2-éthylhexyl(méth)acrylate, le (méth)acrylate de lauryle, le (méth) acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle.

8. Composition selon l'une quelconque des revendications 1 à 7, **caractérisée par le fait que** le polymère siliconé greffé comprend sur la chaîne silicone principale, au moins un groupement organique à caractère anionique obtenu par l'(homo)polymérisation radicalaire d'au moins un monomère anionique de type acide carboxylique insaturé, partiellement ou totalement neutralisé sous la forme d'un sel.

9. Composition selon l'une quelconque des revendications 1 à 8, **caractérisée par le fait que** le polymère siliconé greffé est choisi parmi les polymères siliconés comportant dans leur structure le motif de formule (I) suivant : 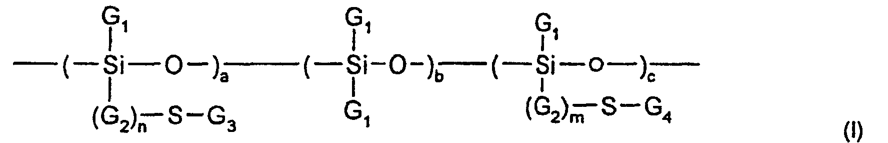 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo) polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

10. Composition selon la revendication 9, **caractérisée par le fait que** le motif de formule (I) présente au moins l'une des caractéristiques suivantes :
- les radicaux G₁ désignent un radical alkyle en C₁-C₁₀;
- n est non nul, et les radicaux G₂ représentent un radical divalent en C₁-C₃ ;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type type (méth)acrylate d'alkyle en C₁-C₁₀.

11. Composition selon la revendication 9 ou 10, **caractérisée par le fait que** le motif de formule (I) présente simultanément les caractéristiques suivantes :
- les radicaux G₁ désignent un radical méthyle;
- n est non nul, et les radicaux G₂ représentent un radical propylène ;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins un monomère du type (méth)acrylate d'isobutyle ou de méthyle.

12. Composition selon l'une quelconque des revendications 1 à 11, **caractérisée par le fait que** la masse moléculaire en nombre du polymère siliconé greffé varie de 10 000 à 1 000 000 environ, et encore plus préférentiellement de 10 000 à 100 000 environ.

13. Composition selon l'une quelconque des revendications 1 à 12, **caractérisée par le fait que** le polymère siliconé greffé est utilisé en une quantité allant de 0,01 à 20% en poids par rapport au poids total de la composition et de préférence de 0,1 à 15% en poids et encore plus préférentiellement de 0,5 à 10 % en poids.

14. Composition selon l'une quelconque des revendications 1 à 13, **caractérisée en ce que** le polymère amphotère est choisi parmi les polymères comportant des motifs A et B répartis statistiquement dans la chaîne polymère et dans lesquels :
- soit A désigne un motif dérivant d'un monomère comportant au moins un atome d'azote basique et B désigne un motif dérivant d'un monomère acide comportant un ou plusieurs groupements carboxyliques ou sulfoniques ;
- soit A et B désignent une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ;
- soit A et B font partie d'une chaîne d'un polymère à motif éthylène α,β-dicarboxylique dont l'un des groupements carboxyliques a été amené à réagir avec une polyamine comportant un ou plusieurs groupements amine primaire ou secondaire ;
- soit A et B peuvent désigner des groupements dérivant de monomères zwittérioniques de carboxybétaïnes ou de sulfobétaïnes.

15. Composition selon la revendication 14, **caractérisée en ce que** les polymères amphotères sont choisis parmi les polymères suivants :
(1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkyl méthacrylates et acrylates, les dialkylaminoalkyl méthacrylamides et acrylamides ;
(2) les polymères dérivés de diallyldialkylammonium et d'au moins un monomère anionique tels que les polymères comportant environ 60 à environ 99% en poids d'unités dérivées d'un monomère de diallyldialkylammonium quaternaire dans lequel les groupements alkyle sont choisis indépendamment parmi les groupements alkyle ayant 1 à 18 atomes de carbone et dans lequel l'anion est dérivé d'un acide ayant une constante d'ionisation supérieure à 10⁻¹³ et 1 à 40% en poids de ce polymère, d'un monomère anionique choisi parmi les acides acryliques ou méthacryliques, le poids moléculaire de ce polymère étant compris entre environ 50 000 et 10 000 000 déterminé par chromatographie par perméation de gel ;
(3) les polymères comportant des motifs dérivant:
a) d'au moins un monomère choisi parmi les acrylamides ou les méthacrylamides substitués sur l'azote par un radical alkyle ;
b) d'au moins un comonomère acide contenant un ou plusieurs groupements carboxyliques réactifs, et
c) au moins un comonomère basique tel que des esters à substituants amine primaire, secondaire, tertiaire et quatemaire des acides acrylique et méthacrylique et le produit de quatemisation du méthacrylate de diméthylaminoéthyle avec le sulfate de diméthyle ou diéthyle ;
(4) les polyamino amides réticulés et alcoylés partiellement ou totalement dérivant de poyaminoamides de formule générale : 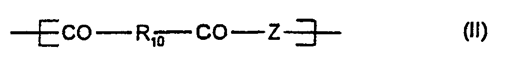 dans laquelle R₁ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalcoylène-polyamine bis-primaire, mono ou bis-secondaire et de préférence représente :
a) dans les proportions de 60 à 100 moles %, le radical 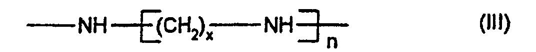 où x=2 et n=2 ou 3 ou bien x=3 et n=2 ce radical dérivant de la diéthylène triamine, de la triéthylène tétraamine ou de la dipropylène triamine ;
b) dans les proportions de 0 à 40 moles % le radical (III) ci-dessus, dans lequel x=2 et n=1 et qui dérive de l'éthylènediamine, ou le radical dérivant de la pipérazine : 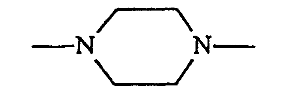
c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels ;
(5) les polymères comportant des motifs zwittérioniques de formule : 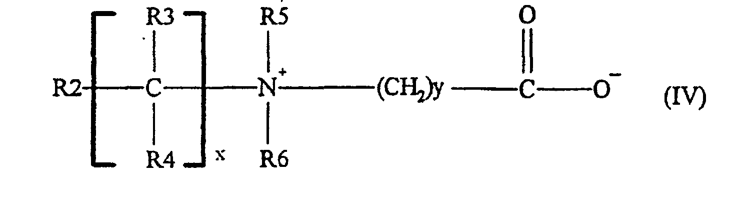 dans laquelle R₂ désigne un groupement insaturé polymérisable tel qu'un groupement acrylate, méthacrylate, acrylamide ou méthacrylamide, x et y représente un nombre entier de 1 à 3, R₃ et R₄ représentent hydrogène, méthyle, éthyle ou propyle, R₅ et R₆ représentent un atome d'hydrogène ou un radical alkyle de telle façon que la somme des atomes de carbone dans R₅ et R₆ ne dépasse pas 10 ; les polymères comprenant de tels motifs pouvant également comporter des motifs dérivés de monomères non zwittérioniques.
(6) les polymères dérivés du chitosane comportant des motifs monomères répondant aux formules suivantes : 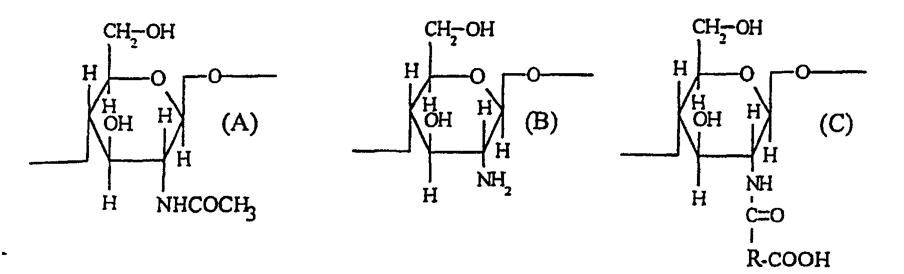 le motif A étant présent dans des proportions comprises entre 0 et 30%, le motif B dans des proportions comprises entre 5 et 50% et le motif C dans des proportions comprises entre 30 et 90%, étant entendu que dans ce motif C, R représente un radical de formule : 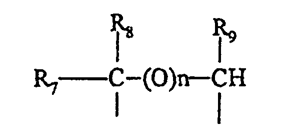 dans laquelle si n=0, R₇, R₈ et R₉ ,identiques ou différents, représentent chacun un atome d'hydrogène, un reste méthyle, hydroxyle, acétoxy, ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, acoylthio, sulfonique, un reste alcoylthio dont le groupe alcoyle porte un reste amino, l'un au moins des radicaux R₇, R₈ et R₉ étant dans ce cas un atome d'hydrogène ;
ou si n=1, R₇, R₈ et R₉ représentent chacun un atome d'hydrogène, ainsi que les sels formés par ces composés avec des bases ou des acides ;
(7) les polymères dérivés de la N-carboxyalkylation du chitosane ;
(8) les polymères répondant à la formule générale (V) : 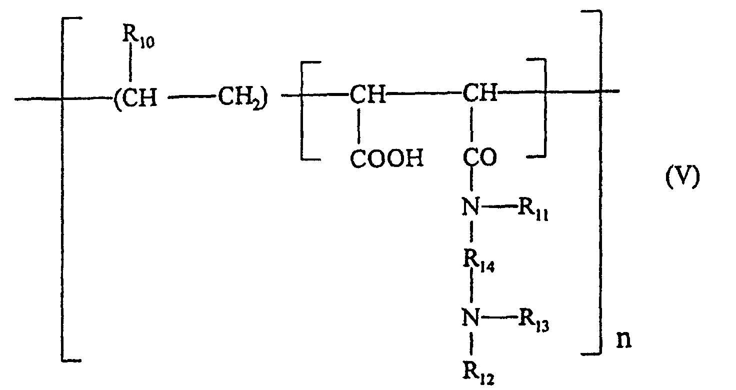 dans laquelle R₁₀ représente un atome d'hydrogène, un radical CH₃O, CH₃CH₂O, phényle, R₁₁ désigne l'hydrogène ou un radical alkyle inférieur tel que méthyle, éthyle, R₁₂ désigne l'hydrogène ou un radical alkyle inférieur tel que méthyle, éthyle, R₁₃ désigne un radical alkyle inférieur tel que méthyle, éthyle ou un radical répondant à la formule : R₁₄-N(R₁₂)₂, R₁₄ représentant un groupement -CH₂-CH₂- , -CH₂-CH₂-CH₂- , -CH₂-CH(CH₃)- , R₁₂ ayant les significations mentionnées ci-dessus, ainsi que les homologues supérieurs de ces radicaux et contenant jusqu'à 6 atomes de carbone ;
(9) les polymères amphotères du type -A-Z-A-Z- choisis parmi:
a) les polymères obtenus par action de l'acide chloracétique ou le chloracétate de sodium sur les composés comportant au moins un motif de formule :
-A-Z-A-Z-A- (VI)
où A désigne un radical 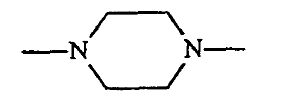 et Z désigne le symbole B ou B', B ou B' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
b) les polymères de formule :
-A-Z-A-Z- (VI')
où A désigne un radical 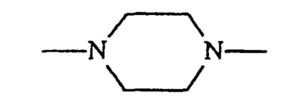 et Z désigne le symbole B ou B' et au moins une fois B'; B ayant la signification indiquée ci-dessus et B' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonction carboxyle ou une ou plusieurs fonctions hydroxyle et bétaïnisées par réaction avec l'acide chloracétique ou du chloracétate de soude ;
(10) les copolymères alkyl (C₁-C₅)vinyléther/anhydride maléique modifié partiellement par semiamidification avec une N,N-dialkylaminoalkylamine telle que la N,N-diméthylaminopropylamine ou par semiestérification avec une N,N-dialcanolamine, ces copolymères comportant éventuellement d'autres comonomères vinyliques tels que le vinylcaprolactame.

16. Composition selon l'une quelconque des revendications 1 à 15, **caractérisée par** le fait le rapport (polymère amphotère)/ (polymère siliconé greffé) est compris entre 0,3 et 8.

17. Composition selon l'une quelconque des revendications 1 à 16, **caractérisée par le fait que** le polymère amphotère est utilisé en une quantité allant de 0, 1 à 20% en poids par rapport au poids total de la composition et de préférence de 0,2 à 15% en poids et encore plus préférentiellement de 0,5 à 10 % en poids.

18. Composition selon l'une quelconque des revendications 1 à 17, **caractérisée par le fait qu'**elle contient en plus au moins un additif choisi dans le groupe constitué par les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

19. Composition selon l'une quelconque des revendications 1 à 18, **caractérisée par le fait que** le milieu cosmétiquement ou dermatologiquement acceptable est constitué par de l'eau ou un mélange d'eau et d'au moins un solvant cosmétiquement acceptable.

20. Composition selon la revendication 19, **caractérisée par le fait que** les solvants cosmétiquement acceptables sont choisis dans le groupe constitué par les monoalcools, les polyalcools, les éthers de glycol, les esters d'acides gras, et leurs mélanges.

21. Composition selon l'une quelconque des revendications 1 à 20, **caractérisée par le fait que** le polymère siliconé greffé est dissous dans le milieu cosmétiquement ou dermatologiquement acceptable ou utilisé sous forme de dispersion aqueuse de particules.

22. Composition selon l'une quelconque des revendications 1 à 21, **caractérisée par le fait que** les matières kératiniques sont des cheveux humains.

23. Composition selon l'une quelconque des revendications 1 à 22, **caractérisée par le fait qu'**elle se présente sous forme de gel, de lait, de crème, de lotion plus ou moins épaissie ou de mousse.

24. Composition selon l'une quelconque des revendications 1 à 23, **caractérisée par le fait qu'**elle est un produit de coiffage.

25. Composition selon l'une quelconque des revendications 1 à 24, **caractérisée par le fait qu'**elle est un produit capillaire choisi dans le groupe constitué par des shampooings ; des produits capillaires à rincer ou non, à appliquer avant ou après un shampooing, une coloration, une décoloration, une permanente ou un défrisage.

26. Composition selon l'une quelconque des revendications 1 à 25, **caractérisée par le fait qu'**elle est conditionnée sous forme de vaporisateur, de flacon pompe ou bien dans un récipient aérosol en vue d'obtenir un spray, une laque ou une mousse.

27. Procédé cosmétique de traitement des matières kératiniques en particulier des cheveux humains, **caractérisé par le fait qu'**il consiste à appliquer sur lesdites matières une composition telle que définie selon l'une quelconque des revendications 1 à 26 puis à effectuer éventuellement un rinçage à l'eau.

## Patentansprüche

1. Kosmetische oder dermatologische Zusammensetzung, die zur Behandlung von Keratinsubstanzen vorgesehen ist, **dadurch gekennzeichnet, daß** sie in einem kosmetisch oder dermatologisch akzeptablen Medium mindestens ein gepfropftes Siliconpolymer mit Polysiloxangerüst, auf das nicht siliconhaltige organische Monomere vom anionischen, amphoteren oder nichtionischen Typ gepfropft sind, und mindestens ein amphoteres Polymer in einem Verhältnis (amphoteres Polymer)/(gepfropftes Siliconpolymer) von 0,25 bis 15 enthält.

2. Zusammensetzung nach Anspruch 1, **dadurch gekennzeichnet, daß** das gepfropfte Siliconpolymer eine Polysiloxanhauptkette aufweist, auf die in der Kette und gegebenenfalls an mindestens einem ihrer Enden mindestens eine organische Gruppe gepfropft ist, die kein Silicon enthält.

3. Zusammensetzung nach Anspruch 1 oder 2, **dadurch gekennzeichnet, daß** das gepfropfte Siliconpolymer durch radikalische Polymerisation mindestens eines nicht siliconhaltigen, anionischen organischen Monomers mit ethylenischer Doppelbindung und/oder eines nicht siliconhaltigen, hydrophoben organischen Monomers mit ethylenischer Doppelbindung und eines Polysiloxans hergestellt werden kann, das in seiner Kette mindestens eine funktionelle Gruppe aufweist, die mit den ethylenischen Doppelbindungen der nicht siliconhaltigen Monomere reagieren kann.

4. Zusammensetzung nach Anspruch 3, **dadurch gekennzeichnet, daß** die anionischen organischen Monomere mit ethylenischer Doppelbindung einzeln oder in Form von Monomerengemischen unter den geradkettigen oder verzweigten, ungesättigten Carbonsäuren ausgewählt sind.

5. Zusammensetzung nach Anspruch 4, **dadurch gekennzeichnet, daß** die anionischen organischen Monomere mit ethylenischer Doppelbindung einzeln oder in Form von Monomerengemischen unter Acrylsäure, Methacrylsäure, Maleinsäure, Maleinsäureanhydrid, Itaconsäure, Fumarsäure, Crotonsäure oder deren Alkali-, Erdalkalioder Ammoniumsalzen oder deren Gemischen ausgewählt sind.

6. Zusammensetzung nach Anspruch 3, **dadurch gekennzeichnet, daß** das hydrophobe organische Monomer mit ethylenischer Doppelbindung einzeln oder in Form von Monomerengemischen unter den Estern von Acrylsäure und einem Alkanol und/oder den Estern von Methacrylsäure und einem Alkanol ausgewählt sind, wobei das Alkanol vorzugsweise 1 bis 18 Kohlenstoffatome aufweist.

7. Zusammensetzung nach Anspruch 6, **dadurch gekennzeichnet, daß** das hydrophobe, organische Monomer mit ethylenischer Doppelbindung einzeln oder in Form von Monomerengemischen unter Isooctyl(meth)acrylat, Isononyl(meth)acrylat, 2-Ethylhexyl(meth)-acrylat, Lauryl(meth)acrylat, Isopentyl(meth)acrylat, n-Butyl(meth)-acrylat, Isobutyl(meth)acrylat, Methyl(meth)acrylat, t-Butyl(meth)-acrylat, Tridecyl(meth)acrylat und Stearyl(meth)acrylat ausgewählt ist.

8. Zusammensetzung nach einem der Ansprüche 1 bis 7, **dadurch gekennzeichnet, daß** das gepfropfte Siliconpolymer in der Siliconhauptkette mindestens eine organische Gruppe mit anionischen Eigenschaften aufweist, die durch radikalische (Homo)polymerisation mindestens eines anionischen Monomers vom Typ einer ungesättigten, zum Teil oder vollständig in Form eines Salzes neutralisierten Carbonsäure hergestellt wird.

9. Zusammensetzung nach einem der Ansprüche 1 bis 8, **dadurch gekennzeichnet, daß** das gepfropfte Siliconpolymer unter den Siliconpolymeren ausgewählt ist, die in ihrer Struktur die Einheit der folgenden Formel (I) enthalten: 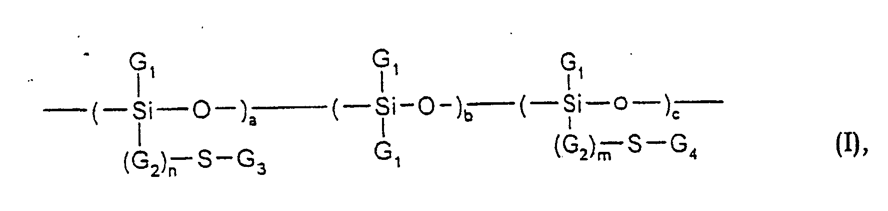 worin die Gruppen G₁, die identisch oder voneinander verschieden sind, Wasserstoff, C₁₋₁₀-Alkyl oder Phenyl; die Gruppen G₂, die identisch oder voneinander verschieden sind, C₁₋₁₀-Alkylen; G₃ eine Polymergruppe, die aus der (Homo)polymerisation mindestens eines anionischen Monomers mit ethylenischer Doppelbindung resultiert; G₄ eine Polymergruppe, die aus der (Homo)polymerisation mindestens eines hydrophoben Monomers mit ethylenischer Doppelbindung resultiert; m und n 0 oder 1; a 0 oder eine ganze Zahl im Bereich von 1 bis 50; b eine ganze Zahl im Bereich von 10 bis 350 und c 0 oder eine ganze Zahl von 1 bis 50 bedeuten, mit der Maßgabe, daß einer der Parameter a oder c von 0 verschieden ist.

10. Zusammensetzung nach Anspruch 9, **dadurch gekennzeichnet, daß** die Einheit der Formel I mindestens eine der folgenden Eigenschaften aufweist:
- die Gruppen G₁ bedeuten eine C₁₋₁₀-Alkylgruppe;
- n ist nicht 0 und die Gruppen G₂ bedeuten eine zweiwertige Gruppe mit 1 bis 3 Kohlenstoffatomen;
- G₃ bedeutet eine Polymergruppe, die bei der (Homo)polymerisation mindestens eines Monomers vom Typ einer Carbonsäure mit ethylenischer Doppelbindung gebildet wird; und
- G₄ bedeutet eine Polymergruppe, die bei der (Homo)polymerisation mindestens eines Monomers vom Typ C₁₋₁₀-Alkyl(meth)acrylat entsteht.

11. Zusammensetzung nach Anspruch 9 oder 10, **dadurch gekennzeichnet, daß** die Einheit der Formel I alle folgenden Eigenschaften aufweist:
- die Gruppen G₁ bedeuten Methyl;
- n ist nicht 0 und die Gruppen G₂ bedeuten Propylen;
- G₃ bedeutet eine Polymergruppe, die bei der (Homo)polymerisation von zumindest Acrylsäure und/oder Methacrylsäure entsteht;
- G₄ bedeutet eine Polymergruppe, die bei der (Homo)polymerisation mindestens eines Monomers vom Typ Isobutyl(meth)acrylat oder Methyl(meth)acrylat entsteht.

12. Zusammensetzung nach einem der Ansprüche 1 bis 11, **dadurch gekennzeichnet, daß** das Zahlenmittel des Molekulargewichts des gepfropften Siliconpolymers im Bereich von etwa 10 000 bis 1 000 000 und noch bevorzugter von etwa 10 000 bis 100 000 liegt.

13. Zusammensetzung nach einem der Ansprüche 1 bis 12, **dadurch gekennzeichnet, daß** das gepfropfte Siliconpolymer in einer Menge von 0,01 bis 20 Gew.-%, bezogen auf das Gesamtgewicht der Zusammensetzung, vorzugsweise 0,1 bis 15 Gew.-% und besonders bevorzugt im Bereich von 0,5 bis 10 Gew.-% verwendet wird.

14. Zusammensetzung nach einem der Ansprüche 1 bis 13, **dadurch gekennzeichnet, daß** das amphotere Polymer unter den Polymeren ausgewählt ist, die in der Polymerkette statistisch verteilte Einheiten A und B enthalten, wobei
- A entweder eine Einheit bezeichnet, die von einem Monomer abgeleitet ist, das mindestens ein basisches Stickstoffatom aufweist, und B eine Einheit bezeichnet, die von einem sauren Monomer abgeleitet ist, das eine oder mehrere Carboxygruppen oder Sulfonsäuregruppen aufweist,
- oder A und B eine kationische Polymerkette bedeuten, die primäre, sekundäre, tertiäre oder quartäre Aminogruppen trägt, wobei mindestens eine der Aminogruppen eine Carboxy- oder Sulfonsäuregruppe trägt, die über eine Kohlenwasserstoffgruppe gebunden ist,
- oder A und B Teil einer α,β-Dicarbonsäure-Ethylen-Polymerkette sind, wobei eine der Carbonsäuregruppen mit einem Polyamin reagieren gelassen wurde, das eine oder mehrere primäre oder sekundäre Aminogruppen aufweist,
- oder A und B Gruppen bedeuten können, die von zwitterionischen Carboxybetain- oder Sulfobetaingruppen stammen.

15. Zusammensetzung nach Anspruch 14, **dadurch gekennzeichnet, daß** die amphoteren Polymere unter den folgenden Polymeren ausgewählt sind:
(1) Den Polymeren, die bei der Copolymerisation eines Monomers, das von einer Vinylverbindung abgeleitet ist, die eine Carboxygruppe trägt, wie insbesondere Acrylsäure, Methacrylsäure, Maleinsäure und α-Chloracrylsäure, und eines basischen Monomers entstehen, das von einer substituierten Vinylverbindung abgeleitet ist, die mindestens ein basisches Atom enthält, wie insbesondere Dialkylaminoalkylmethacrylate und -acrylate, Dialkylaminoalkylmethacrylamide und Dialkylaminoalkylacrylamide;
(2) Die von Diallyldialkylammonium und mindestens einem anionischen Monomer abgeleiteten Polymere, wie insbesondere die Polymere, die etwa 60 bis etwa 99 Gew.-% von einem quartären Diallyldialkylammoniummonomer abgeleitete Einheiten, wobei die Alkylgruppen unabhängig voneinander unter den Alkylgruppen mit 1 bis 18 Kohlenstoffatomen ausgewählt sind und das Anion von einer Säure angeleitet ist, die eine Ionisierungskonstante über 10⁻¹³ aufweist, und 1 bis 40 Gew.-% eines anionischen Monomers enthalten, das unter Acrylsäure und Methacrylsäure ausgewählt ist, wobei das mit Gelpermeationschromatographie bestimmte Molekulargewicht des Polymers im Bereich von etwa 50 000 bis 10 000 000 liegt;
(3) Den Polymeren, die Einheiten enthalten, die abgeleitet sind von:
a) mindestens einem Monomer, das unter den Acrylamiden oder Methyacrylamiden, die am Stickstoffatom mit einer Alkylgruppe substituiert sind, ausgewählt ist,
b) mindestens einem sauren Comonomer, das eine oder mehrere reaktive Carboxygruppen enthält, und
c) mindestens einem basischen Comonomer, wie den Estern von Acrylsäure und Methacrylsäure mit primären, sekundären, tertiären und quaternären Aminogruppen als Substituenten, und dem Produkt der Quaternisierung von Dimethylaminoethylmethacrylat mit Dimethyl- oder Diethylsulfat.
(4) Den Polyaminoamiden, die ganz oder teilweise vernetzt und alkyliert sind und von Polyaminoamiden der folgenden Formel abgeleitet sind: 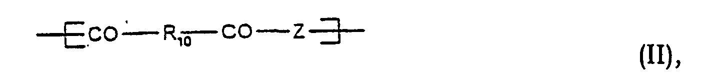 worin R₁₀ eine zweiwertige Gruppe bedeutet, die von einer gesättigten Dicarbonsäure, einer aliphatischen Mono- oder Dicarbonsäure mit ethylenischer Doppelbindung, einem Ester dieser Säuren mit einem niederen Alkanol mit 1 bis 6 Kohlenstoffatomen oder einer Gruppe, die aus der Umsetzung einer dieser Säuren mit einem bisprimären oder bis-sekundären Amin stammt, abgeleitet ist; und Z eine bis-primäre, mono oder bis-sekundäre Polyalkylen-Polyamin-Gruppe bedeutet und vorzugsweise:
a) in Anteilen von 60 bis 100 Mol-% die Gruppe 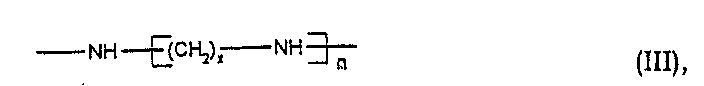 worin bedeuten:
x = 2 und n = 2 oder 3 oder x = 3 und n = 2, wobei diese Gruppe von Diethylentriamin, Triethylentetramin oder Dipropylentriamin abgeleitet ist;
b) in Anteilen von 0 bis 40 Mol-% die oben genannte Gruppe (III), worin x = 2 und n = 1, die von Ethylendiamin abgeleitet ist, oder die von Piperazin stammende Gruppe: 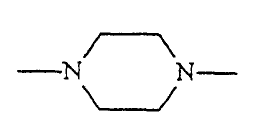
c) in Anteilen von 0 bis 20 Mol-% die Gruppe -NH-(CH₂)₆-NH-, die von Hexylendiamin stammt,
wobei diese Polyaminoamide durch die Zugabe eines bifunktionellen Vernetzungsmittels, das unter den Epihalohydrinen, Diepoxiden, Dianhydriden und zweifach ungesättigten Derivaten ausgewählt ist, in einer Menge von 0,025 bis 0,35 mol Vernetzungsmittel pro Aminogruppe des Polyaminoamids vernetzt und durch Einwirkung von Acrylsäure, Chloressigsäure, eines Alkansultons oder deren Salzen alkyliert sind;
(5) Den Polymeren mit zwitterionischen Einheiten der folgenden Formel: 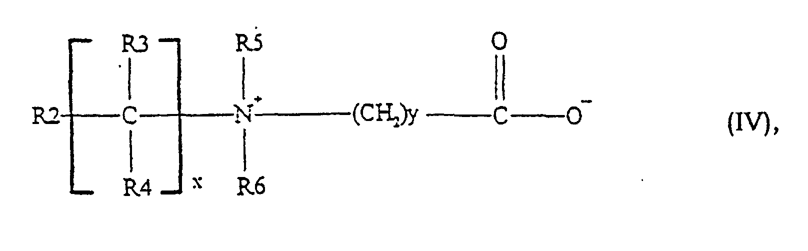 worin R₂ eine ungesättigte, polymerisierbare Gruppe, wie Acrylat, Methacrylat, Acrylamid oder Methacrylamid, x und y eine ganze Zahl im Bereich von 1 bis 3, R₃ und R₄ Wasserstoff, Methyl, Ethyl oder Propyl, R₅ und R₆ Wasserstoff oder Alkyl bedeuten, mit der Maßgabe, daß die Summe der Kohlenstoffatome in den Gruppen R₅ und R₆ 10 nicht übersteigt; die Polymere, die diese Einheiten enthalten, können ferner Einheiten aufweisen, die von nicht zwitterionischen Monomeren abgeleitet sind;
(6) Den von Chitosan abgeleiteten Polymeren, die Monomereinheiten der folgenden Formeln enthalten: 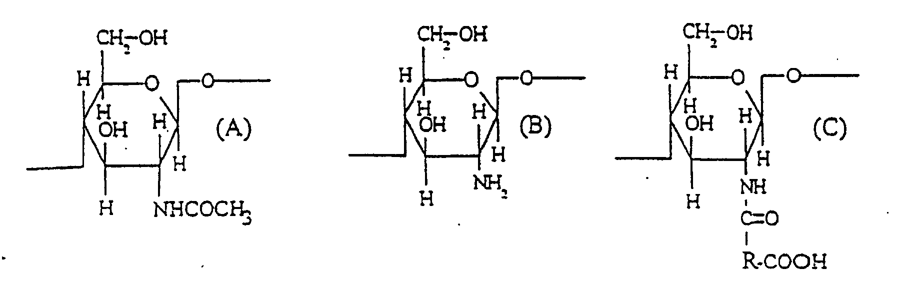 wobei die Einheit A in Anteilen von 0 bis 30 %, die Einheit B in Anteilen von 5 bis 50 % und die Einheit C in Anteilen von 30 bis 90 % vorliegt, mit der Maßgabe, daß in der Einheit C die Gruppe R die folgende Gruppe bedeutet: 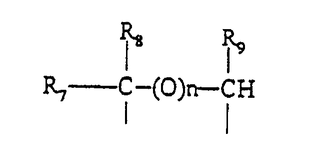 worin, wenn n = 0 ist, die Gruppen R₇, R₈ und R₉, die identisch oder voneinander verschieden sind, Wasserstoff, Methyl, Hydroxy, Acetoxy, Amino, eine Monoalkylamino- oder Dialkylaminogruppe, die gegebenenfalls durch ein oder mehrere Stickstoffatome unterbrochen und/oder gegebenenfalls mit einer oder mehreren Amino- Hydroxy-, Carboxy-, Alkylthio- oder Sulfonsäuregruppen substituiert sind, oder eine Alkylthiogruppe, deren Alkylgruppe eine Aminogruppe trägt, bedeuten, wobei in diesem Fall mindestens eine der Gruppen R₇, R₈ und R₉ Wasserstoff bedeutet;
oder, wenn n = 1 ist, die Gruppen R₁₇, R₁₈ und R₁₉ Wasserstoff bedeuten,
sowie den Salzen, die diese Verbindungen mit Basen oder Säuren bilden.
(7) Den Polymeren, die von N-carboxyalkyliertem Chitosan abgeleitet sind;
(8) Den Polymeren, die der allgemeinen Formel (V) entsprechen: 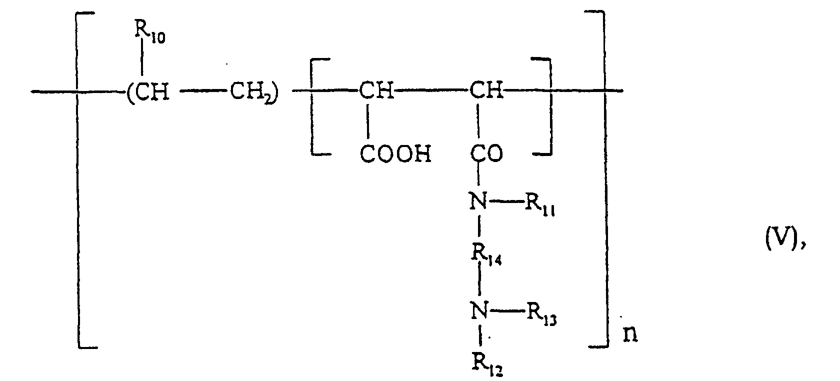 worin bedeuten: R₁₀ Wasserstoff, CH₃O, CH₃CH₂O oder Phenyl, R₁₁ Wasserstoff oder eine niedere Alkylgruppe, wie Methyl und Ethyl, R₁₂ Wasserstoff oder eine niedere Alkylgruppe, wie Methyl und Ethyl, und R₁₃ eine niedere Alkylgruppe, wie Methyl und Ethyl, oder eine Gruppe der Formel R₁₄-N(R₁₂)₂, wobei R₁₄ -CH₂-CH₂-, -CH₂-CH₂-CH₂- oder -CH₂-CH(CH₃)- bedeutet und R₁₂ die oben angegebenen Bedeutungen aufweist,
sowie die höheren Homologen dieser Gruppen, die bis zu 6 Kohlenstoffatome enthalten.
(9) Den amphoteren Polymeren vom Typ -A-Z-A-Z-, die ausgewählt sind unter:
a) den Polymeren, die durch Einwirkung von Chloressigsäure oder Natriumchloracetat auf Verbindungen hergestellt sind, welche mindestens eine Einheit der folgenden Formel aufweisen:
-A-Z-A-Z-A- (VI),
wobei A die Gruppe 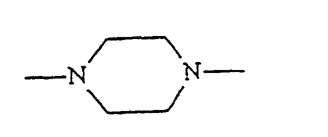 und Z B oder B' bedeutet, wobei B und B', die identisch oder voneinander verschieden sind, eine zweiwertige. Gruppe bedeuten, die eine geradkettige oder verzweigte Alkylengruppe mit bis zu 7 Kohlenstoffatomen in der Hauptkette ist, die unsubstituiert vorliegt oder mit Hydroxy substituiert ist und ferner Sauerstoff-, Stickstoffoder Schwefelatome und 1 bis 3 aromatische und/oder heterocyclische Ringe enthalten kann, wobei die Sauerstoff-, Stickstoff- und Schwefelatome in der Form folgender Gruppen vorliegen: Ether, Thioether, Sulfoxid, Sulfon, Sulfonium, Alkylamin, Alkenylamin, Hydroxy, Benzylamin, Aminoxid, quaternäres Ammonium, Amid, Imid, Alkohol, Ester und/oder Urethan.
b) den Polymeren der Formel
-A-Z-A-Z- (VI'),
wobei A die Gruppe 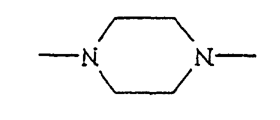 und Z B oder B' und mindestens einmal B' bedeutet, wobei B die oben angegebene Bedeutung aufweist und B' eine zweiwertige Gruppe bedeutet, die eine geradkettige oder verzweigte Alkylengruppe mit bis zu 7 Kohlenstoffatomen in der Hauptkette ist, die unsubstituiert vorliegt oder ein- oder mehrfach mit Hydroxy substituiert ist und ein oder mehrere Stickstoffatome aufweist, wobei das Stickstoffatom mit einer Alkylgruppe substituiert ist, die gegebenenfalls durch Sauerstoff unterbrochen ist, zwingend eine oder mehrere Carboxygruppen oder eine oder mehrere Hydroxygruppen enthält und durch Umsetzung mit Chloressigsäure oder Natriumchloracetat in das Betain überführt ist.
(10) Den Copolymeren C₁₋₅-Alkylvinylether / Maleinsäureanhydrid, das partiell durch Semiamidierung mit einem N,N-Dialkylaminoalkylamin, wie N,N-Dimethylaminopropylamin, oder Semiveresterung mit einem N,N-Dialkanolamin modifiziert wurde; diese Copolymere können ferner weitere Vinylcomonomere, wie Vinylcaprolactam, enthalten.

16. Zusammensetzung nach einem der Ansprüche 1 bis 15, **dadurch gekennzeichnet, daß** das Verhältnis (amphoteres Polymer)/(gepfropftes Siliconpolymer) im Bereich von 0,3 bis 8 liegt.

17. Zusammensetzung nach einem der Ansprüche 1 bis 16, **dadurch gekennzeichnet, daß** das amphotere Polymer in einer Menge von 0,1 bis 20 Gew.-%, bezogen auf das Gesamtgewicht der Zusammensetzung, vorzugsweise 0,2 bis 15 Gew.-% und noch bevorzugter 0,5 bis 10 Gew.-% verwendet wird.

18. Zusammensetzung nach einem der Ansprüche 1 bis 17, **dadurch gekennzeichnet, daß** sie ferner mindestens einen Zusatzstoff enthält, der unter den Verdickungsmitteln, Fettsäureestern, Estern von Fettsäuren und Glycerin, Siliconen, grenzflächenaktiven Stoffen, Parfums, Konservierungsmitteln, Sonnenschutzfiltern, Proteinen, Vitaminen, Polymeren, pflanzlichen Ölen, tierischen Ölen, Mineralölen oder synthetischen Ölen oder beliebigen herkömmlich in der Kosmetik verwendeten Zusatzstoffen ausgewählt ist.

19. Zusammensetzung nach einem der Ansprüche 1 bis 18, **dadurch gekennzeichnet, daß** das kosmetisch oder dermatologisch akzeptable Medium aus Wasser oder einem Gemisch von Wasser und mindestens einem kosmetisch akzeptablen Lösungsmittel besteht.

20. Zusammensetzung nach Anspruch 19, **dadurch gekennzeichnet, daß** die kosmetisch akzeptablen Lösungsmittel unter den Monoalkoholen, Polyalkoholen, Glykolethern, Fettsäureestern und deren Gemischen ausgewählt sind.

21. Zusammensetzung nach einem der Ansprüche 1 bis 20, **dadurch gekennzeichnet, daß** das gepfropfte Siliconpolymer in dem kosmetisch oder dermatologisch akzeptablen Medium gelöst ist oder in Form einer wäßrigen Dispersion von Partikeln verwendet wird.

22. Zusammensetzung nach einem der Ansprüche 1 bis 21, **dadurch gekennzeichnet, daß** es sich bei den Keratinsubstanzen um menschliches Haar handelt.

23. Zusammensetzung nach einem der Ansprüche 1 bis 22, **dadurch gekennzeichnet, daß** sie als Gel, Milch, Creme, mehr oder weniger dickflüssige Lotion oder Schaum vorliegt.

24. Zusammensetzung nach einem der Ansprüche 1 bis 23, **dadurch gekennzeichnet, daß** es sich um ein Produkt zum Frisieren handelt.

25. Zusammensetzung nach einem der Ansprüche 1 bis 24, **dadurch gekennzeichnet, daß** es sich um ein Produkt für das Haar handelt, das unter den Haarwaschmitteln und Produkten für das Haar, die ausgespült oder nicht ausgespült werden und vor oder nach einer Haarwäsche, Färbung, Entfärbung, Dauerwelle oder Entkräuselung aufgetragen werden, ausgewählt ist.

26. Zusammensetzung nach einem der Ansprüche 1 bis 25, **dadurch gekennzeichnet, daß** sie zur Bildung eines Sprays, eines Lacks oder eines Schaums in einem Zerstäuber, Pumpflakon oder Aersolbehälter konfektioniert ist.

27. Verfahren zur Behandlung von Keratinsubstanzen und insbesondere menschlichem Haar, **dadurch gekennzeichnet, daß** es darin besteht, auf die Keratinsubstanzen eine Zusammensetzung nach einem der Ansprüche 1 bis 26 aufzutragen und gegebenenfalls mit Wasser zu spülen.

## Claims

1. Cosmetic or dermatological composition intended for treating keratin substances, **characterized in that** it comprises, in a cosmetically or dermatologically acceptable medium, at least one grafted silicone polymer containing a polysiloxane skeleton grafted with non-silicone organic monomers of the anionic, amphoteric or nonionic type and at least one amphoteric polymer, in an (amphoteric polymer)/(grafted silicone polymer) ratio of between 0.25 and 15.

2. Composition according to Claim 1, **characterized in that** the grafted silicone polymer comprises a main polysiloxane chain on which is grafted, inside the said chain as well as, optionally, on at least one of its ends, at least one organic group containing no silicone.

3. Composition according to Claim 1 or 2, **characterized in that** the grafted silicone polymer can be obtained by radical copolymerization between, on the one hand, at least one non-silicone anionic organic monomer having ethylenic unsaturation and/or a non-silicone hydrophobic organic monomer having ethylenic unsaturation, and, on the other hand, a polysiloxane having in its chain at least one functional group capable of reacting with the said ethylenic unsaturations of the said non-silicone monomers.

4. Composition according to Claim 3, **characterized in that** the anionic organic monomer containing ethylenic unsaturation is chosen, alone or in the form of a monomer mixture, from linear or branched unsaturated carboxylic acids.

5. Composition according to Claim 4, **characterized in that** the anionic organic monomer containing ethylenic unsaturation is chosen, alone or in the form of a monomer mixture, from acrylic acid, methacrylic acid, maleic acid, maleic anhydride, itaconic acid, fumaric acid and crotonic acid, or the alkali-metal, alkaline-earth metal or ammonium salts thereof, or mixtures thereof.

6. Composition according to Claim 3, **characterized in that** the hydrophobic organic monomer containing ethylenic unsaturation is chosen, alone or as a monomer mixture, from acrylic acid esters of an alkanol and/or methacrylic acid esters of an alkanol, the alkanol preferably being C₁-C₁₈.

7. Composition according to Claim 6, **characterized in that** the hydrophobic organic monomer containing ethylenic unsaturation is chosen, alone or as a monomer mixture, from the group consisting of isooctyl (meth)acrylate, isononyl (meth)acrylate, 2-ethylhexyl (meth)acrylate, lauryl (meth)acrylate, isopentyl (meth)acrylate, n-butyl (meth)acrylate, isobutyl (meth)acrylate, methyl (meth)acrylate, tert-butyl (meth)acrylate, tridecyl (meth)acrylate and stearyl (meth)acrylate.

8. Composition according to any one of Claims 1 to 7, **characterized in that** the grafted silicone polymer comprises, on the main silicone chain, at least one organic group of anionic nature obtained by radical (homo)polymerization of at least one anionic monomer of unsaturated carboxylic acid type, partially or totally neutralized in the form of a salt.

9. Composition according to any one of Claims 1 to 8, **characterized in that** the grafted silicone polymer is chosen from silicone polymers containing in their structure the unit of formula (I) below: 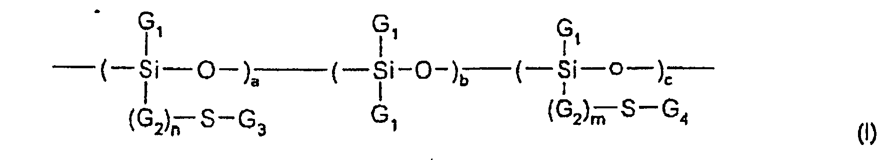 in which the radicals G₁, which may be identical or different, represent hydrogen or a C₁-C₁₀ alkyl radical or alternatively a phenyl radical; the radicals G₂, which may be identical or different, represent a C₁-C₁₀ alkylene group; G₃ represents a polymer residue resulting from the (homo)polymerization of at least one anionic monomer containing ethylenic unsaturation; G₄ represents a polymer residue resulting from the (homo)polymerization of at least one hydrophobic monomer containing ethylenic unsaturation; m and n are equal to 0 or 1; a is an integer ranging from 0 to 50; b is an integer which may be between 10 and 350, c is an integer ranging from 0 to 50; with the proviso that one of the parameters a and c is other than 0.

10. Composition according to Claim 9, **characterized in that** the unit of formula (I) has at least one of the following characteristics:
- the radicals G₁ denote a C₁-C₁₀ alkyl radical;
- n is non-zero and the radicals G₂ represent a divalent C₁-C₃ radical;
- G₃ represents a polymeric radical resulting from the (homo)polymerization of at least one monomer of the carboxylic acid type containing ethylenic unsaturation;
- G₄ represents a polymeric radical resulting from the (homo)polymerization of at least one monomer of the C₁-C₁₀ alkyl (meth)acrylate type.

11. Composition according to Claim 9 or 10, **characterized in that** the unit of formula (I) simultaneously has the following characteristics:
- the radicals G₁ denote a methyl radical;
- n is non-zero and the radicals G₂ represent a propylene radical;
- G₃ represents a polymeric radical resulting from the (homo)polymerization of at least one acrylic acid and/or methacrylic acid;
- G₄ represents a polymeric radical resulting from the (homo)polymerization of at least one monomer of the isobutyl or methyl (meth)acrylate type.

12. Composition according to any one of Claims 1 to 11, **characterized in that** the number-average molecular mass of the grafted silicone polymer ranges approximately from 10,000 to 1,000,000 and even more preferably approximately from 10,000 to 100,000.

13. Composition according to any one of Claims 1 to 12, **characterized in that** the grafted silicone polymer is used in an amount ranging from 0.01 to 20% by weight relative to the total weight of the composition and preferably from 0.1 to 15% by weight and even more preferably from 0.5 to 10% by weight.

14. Composition according to any one of Claims 1 to 13, **characterized in that** the amphoteric polymer is chosen from polymers containing units A and B randomly distributed in the polymer chain and in which:
- either A denotes a unit derived from a monomer containing at least one basic nitrogen atom and B denotes a unit derived from an acidic monomer containing one or more carboxylic or sulphonic groups;
- or A and B denote a cationic polymer chain containing primary, secondary, tertiary or quaternary amine groups in which at least one of the amine groups bears a carboxylic or sulphonic group connected via a hydrocarbon radical;
- or A and B form part of a polymer chain containing an α,β-dicarboxylic ethylene unit in which one of the carboxylic groups has been reacted with a polyamine containing one or more primary or secondary amine groups;
- or A and B can denote groups derived from carboxybetaine or sulphobetaine zwitterionic monomers.

15. Composition according to Claim 14, **characterized in that** the amphoteric polymers are chosen from the following polymers:
(1) polymers resulting from the copolymerization of a monomer derived from a vinyl compound bearing a carboxylic group such as, more particularly, acrylic acid, methacrylic acid, maleic acid or alpha-chloroacrylic acid, and of a basic monomer derived from a substituted vinyl compound containing at least one basic atom such as, more particularly, dialkylaminoalkyl methacrylates and acrylates, dialkylaminoalkyl methacrylamides and acrylamides;
(2) polymers derived from diallyldialkylammonium and from at least one anionic monomer, such as polymers containing from about 60 to about 99% by weight of units derived from a quaternary diallyldialkylammonium monomer in which the alkyl groups are chosen, independently, from alkyl groups having 1 to 18 carbon atoms and in which the anion is derived from an acid having an ionization constant of greater than 10⁻¹³ and 1 to 40% by weight of this polymer, of an anionic monomer chosen from acrylic or methacrylic acid, the molecular weight of this polymer being approximately between 50,000 and 10,000,000, determined by gel permeation chromatography;
(3) polymers containing units derived:
a) from at least one monomer chosen from acrylamides or methacrylamides substituted on the nitrogen with an alkyl radical,
b) from at least one acidic comonomer containing one or more reactive carboxylic groups, and
c) from at least one basic comonomer such as esters containing primary, secondary, tertiary and quaternary amine substituents of acrylic acid and methacrylic acid and the quaternization product of dimethylaminoethyl methacrylate with dimethyl or diethyl sulphate;
(4) partially or totally alkylated and crosslinked polyamino amides derived from polyamino amides of general formula: 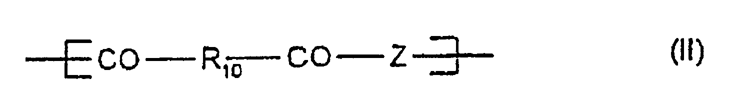 in which R₁₀ represents a divalent radical derived from a saturated dicarboxylic acid, from an aliphatic mono-or dicarboxylic acid containing an ethylenic double bond, from an ester of a lower alkanol having 1 to 6 carbon atoms of these acids or from a radical derived from the addition of any one of the said acids to a bis-primary or bis-secondary amine, and Z denotes a radical of a bis-primary, mono- or bis-secondary polyalkylene polyamine and preferably represents:
a) in proportions of 60 to 100 mol%, the radical 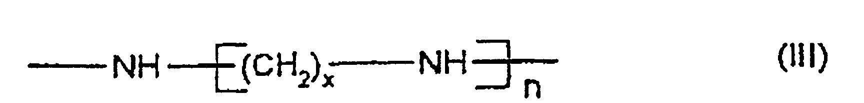 where x=2 and n=2 or 3 or alternatively x=3 and n=2 this radical being derived from diethylenetriamine, triethylenetetraamine or dipropylenetriamine;
b) in proportions of 0 to 40 mol%, the radical (III) above, in which x=2 and n=1 and which is derived from ethylenediamine, or the radical derived from piperazine: 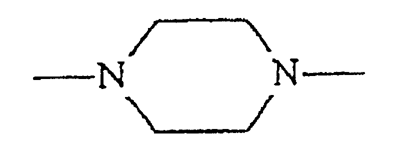
c) in proportions of 0 to 20 mol%, the radical -NH-(CH₂)₆-NH- derived from hexamethylenediamine, these polyamino amides being crosslinked by addition of a difunctional crosslinking agent chosen from epihalohydrins, diepoxides, dianhydrides and bis-unsaturated derivatives, using from 0.025 to 0.35 mol of crosslinking agent per amine group of the polyamino amide and alkylated by the action of acrylic acid, chloroacetic acid or an alkane sultone, or salts thereof;
(5) polymers containing zwitterionic units of formula: 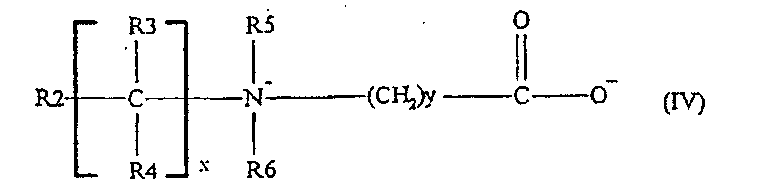 in which R₂ denotes a polymerizable unsaturated group such as an acrylate, methacrylate, acrylamide or methacrylamide group, x and y represent an integer from 1 to 3, R₃ and R₄ represent hydrogen, methyl, ethyl or propyl, R₅ and R₆ represent a hydrogen atom or an alkyl radical such that the sum of the carbon atoms in R₅ and R₆ does not exceed 10; it also being possible for the polymers comprising such units to contain units derived from non-zwitterionic monomers;
(6) chitosan-derived polymers containing monomer units corresponding to the following formulae: 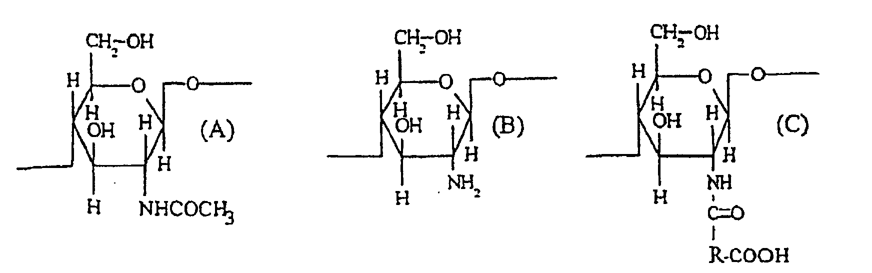 the unit A being present in proportions of between 0 and 30%, the unit B in proportions of between 5 and 50% and the unit C in proportions of between 30 and 90%, it being understood that in this unit C, R represents a radical of formula: 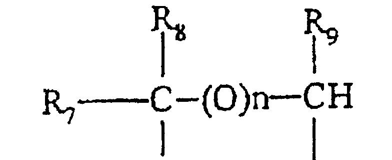 in which if n=0, R₇, R₈ and R₉, which may be identical or different, each represent a hydrogen atom, a methyl, hydroxyl, acetoxy or amino residue, a monoalkylamine residue or a dialkylamine residue optionally interrupted by one or more nitrogen atoms and/or optionally substituted by one or more amine, hydroxyl, carboxyl, alkylthio or sulphonic groups, an alkylthio residue in which the alkyl group bears an amino residue, at least one of the radicals R₇, R₈ and R₉ being, in this case, a hydrogen atom;
or if n=1, R₇, R₈ and R₉ each represent a hydrogen atom, as well as the salts formed by these compounds with acids or bases;
(7) polymers derived from the N-carboxyalkylation of chitosan;
(8) polymers corresponding to the general formula (V), 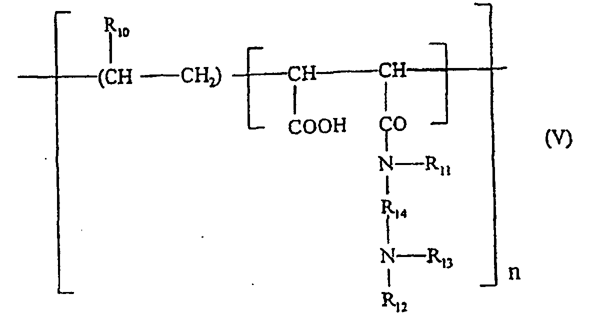 in which R₁₀ represents a hydrogen atom or a CH₃O, CH₃CH₂O or phenyl radical, R₁₁ denotes hydrogen or a lower alkyl radical such as methyl or ethyl, R₁₂ denotes hydrogen or a lower alkyl radical such as methyl or ethyl, R₁₃ denotes a lower alkyl radical such as methyl or ethyl or a radical corresponding to the formula: R₁₄-N(R₁₂)₂, R₁₄ representing a -CH₂-CH₂-, -CH₂-CH₂-CH₂- or -CH₂-CH(CH₃)- group, R₁₂ having the meanings given above, as well as the higher homologues of these radicals and containing up to 6 carbon atoms;
(9) amphoteric polymers of the type -A-Z-A-Z, chosen from:
a) polymers obtained by the action of chloroacetic acid or sodium chloroacetate on compounds containing at least one unit of formula:
-A-Z-A-Z-A- (VI)
where A denotes a radical 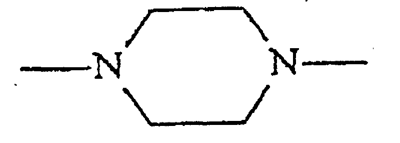 and Z denotes the symbol B or B', B and B', which may be identical or different, denote a divalent radical which is an alkylene radical with a straight or branched chain containing up to 7 carbon atoms in the main chain, unsubstituted or substituted with hydroxyl groups and also being able to contain oxygen, nitrogen or sulphur atoms, and 1 to 3 aromatic and/or heterocyclic rings, the oxygen, nitrogen and sulphur atoms being present in the form of ether, thioether, sulphoxide, sulphone, sulphonium, alkylamine or alkenylamine groups, and hydroxyl, benzylamine, amine oxide, quaternary ammonium, amide, imide, alcohol, ester and/or urethane groups;
b) polymers of formula:
-A-Z-A-Z- (VI')
where A denotes a radical 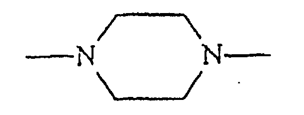 and Z denotes the symbol B or B' and at least once B', B having the meaning given above and B' being a divalent radical which is an alkylene radical containing a straight or branched chain having up to 7 carbon atoms in the main chain, unsubstituted or substituted with one or more hydroxyl radicals and containing one or more nitrogen atoms, the nitrogen atom being substituted with an alkyl chain optionally interrupted by an oxygen atom and necessarily containing one or more carboxyl functions or one or more hydroxyl functions and betainized by reaction with chloroacetic acid or sodium chloroacetate;
(10) copolymers of (C₁-C₅)alkyl vinyl ether/maleic anhydride partially modified by semiamidification with an N,N-dialkylaminoalkylamine such as N,N-dimethylaminopropylamine, or by semiesterification with an N,N-dialkanolamine, these copolymers also optionally containing other vinylic comonomers such as vinylcaprolactam.

16. Composition according to any one of Claims 1 to 15, **characterized in that** the (amphoteric polymer)/(grafted silicone polymer) ratio is between 0.3 and 8.

17. Composition according to any one of Claims 1 to 16, **characterized in that** the amphoteric polymer is used in an amount ranging from 0.1 to 20% by weight relative to the total weight of the composition, and preferably from 0.2 to 15% by weight and even more preferably from 0.5 to 10% by weight.

18. Composition according to any one of Claims 1 to 17, **characterized in that** it also contains at least one additive chosen from the group consisting of thickeners, fatty acid esters, fatty acid esters of glycerol, silicones, surfactants, fragrances, preserving agents, sunscreens, proteins, vitamins, polymers, plant, animal, mineral or synthetic oils or any other additive conventionally used in the cosmetic field.

19. Composition according to any one of Claims 1 to 18, **characterized in that** the cosmetically or dermatologically acceptable medium consists of water or a mixture of water and at least one cosmetically acceptable solvent.

20. Composition according to Claim 19, **characterized in that** the cosmetically acceptable solvents are chosen from the group consisting of monoalcohols, polyalcohols, glycol ethers and fatty acid esters, and mixtures thereof.

21. Composition according to any one of Claims 1 to 20, **characterized in that** the grafted silicone polymer is dissolved in the cosmetically or dermatologically acceptable medium or is used in the form of an aqueous dispersion of particles.

22. Composition according to any one of Claims 1 to 21, **characterized in that** the keratin substance is human hair.

23. Composition according to any one of Claims 1 to 22, **characterized in that** it is in the form of a gel, a milk, a cream, a relatively thickened lotion or a mousse.

24. Composition according to any one of Claims 1 to 23, **characterized in that** it is a styling product.

25. Composition according to any one of Claims 1 to 24, **characterized in that** it is a hair product chosen from the group consisting of shampoos, rinse-out or leave-in hair products, to be applied before or after shampooing, dyeing, bleaching, permanent-waving or straightening the hair.

26. Composition according to any one of Claims 1 to 25, **characterized in that** it is packaged in the form of a vaporizer, a pump-dispenser bottle or in an aerosol container in order to obtain a spray, a lacquer or a mousse.

27. Cosmetic process for treating keratin substances, in particular human hair, **characterized in that** it consists in applying a composition as defined according to any one of Claims 1 to 26 to the said substances, and then optionally in rinsing with water.
